(19) Europäisches Patentamt
European Patent Office
Office européen des brevets

(11) EP 4 752 219 A2

(12) EUROPEAN PATENT APPLICATION

(43) Date of publication:
**03.06.2026 Bulletin 2026/23**

(21) Application number: **25208549.3**

(22) Date of filing: **14.10.2025**

(51) International Patent Classification (IPC):
***C12N 9/12*** *(2006.01)* ***C12P 19/34*** *(2006.01)*

(52) Cooperative Patent Classification (CPC):
**C12N 9/1247; C12P 19/34**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **14.10.2024 CN 202411429549**

(71) Applicant: **Nanjing Vazyme Biotech Co., Ltd.**
**Nanjing, Jiangsu 210046 (CN)**

(72) Inventors:
- **HE, Wei**
**JIANGSU, 210046 (CN)**
- **JIN, Qiuheng**
**JIANGSU, 210046 (CN)**
- **LI, Ji**
**JIANGSU, 210046 (CN)**
- **XU, Xiaoyu**
**JIANGSU, 210046 (CN)**
- **YUAN, Zheng**
**JIANGSU, 210046 (CN)**
- **ZHOU, Hongli**
**JIANGSU, 210046 (CN)**

(74) Representative: **Ostertag & Partner Patentanwälte mbB**
**Azenbergstraße 35**
**70174 Stuttgart (DE)**

Remarks:
The complete document including Reference Table(s) and the Sequence Listing(s) can be downloaded from the EPO website

# (54) RNA POLYMERASE VARIANT AND USE THEREOF

(57) The present application provides a class of T7 RNA polymerase variants and the use thereof, wherein the amino acid sequences of the T7 RNA polymerase variants comprise, relative to SEQ ID NO: 1, at least one substitution or deletion at amino acid positions selected from R34, K172, Y178, R386, D388, Q435, N437 or D438. The variants have improved catalytic activity relative to the wild-type T7 RNA polymerase, which can increase the capping rate of mRNA products during in-vitro transcription (co-transcriptional capping). In addition, the present application further provides a method for preparing RNA by using such variants. By adopting the method to prepare RNA molecules, more capped mRNAs can be obtained.

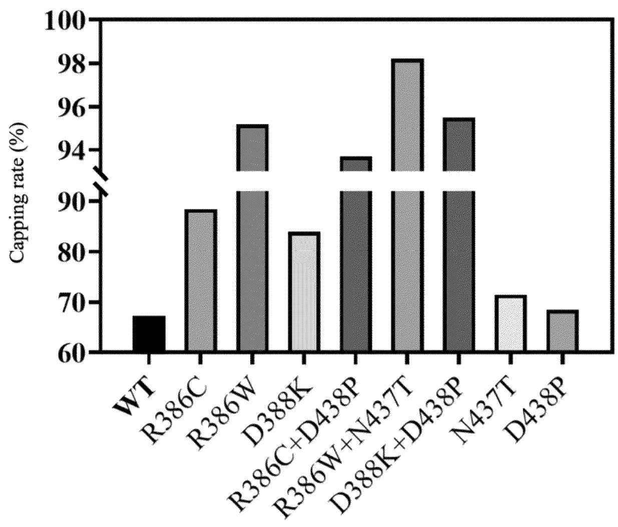


FIG. 5



Processed by Luminess, 75001 PARIS (FR)

## Description

### Technical Field

The present application relates to the field of biotechnology, and in particular relates to an RNA polymerase variant, and a preparation method therefor, and the use thereof in RNA synthesis.

### Background Art

A 7-methylguanosine (m7G) cap exists at the 5' end of an intact eukaryotic mRNA, which is formed in vivo by the catalysis of RNA triphosphatase, mRNA guanylyltransferase, mRNA methyltransferase, and mRNA nucleoside 2'-O-methyltransferase. The 5' cap structure is involved in processes such as preventing mRNA degradation by exonucleases, reducing mRNA immunogenicity, adjusting mRNA half-life and regulating translation. In the process of preparing mRNA by in-vitro transcription (IVT), researchers typically add cap analogues during the preparation to mimic the 5' cap structure of the eukaryotic cellular mRNA.

The cap structure at the 5' end of mRNA has a significant impact on mRNA in terms of stability, translation efficiency, immunogenicity, etc. With the continuous advancement of research, the importance of cap analogues has become increasingly evident. Consequently, novel cap analogues have been continuously developed for use in mRNA vaccines and therapeutic RNA. Nowadays, cap analogues have evolved to the third generation. The first-generation cap analogues, due to the presence of two free 3'-OH groups, tend to be incorporated in a reverse direction. Thus, the first-generation cap analogues are rarely seen on the market. The cap analogues commonly seen on the market mainly include the second-generation cap analogues, ARCA (Formula 1) and the third-generation cap analogues (such as Formula 2), with main structural formulas exemplified as follows:

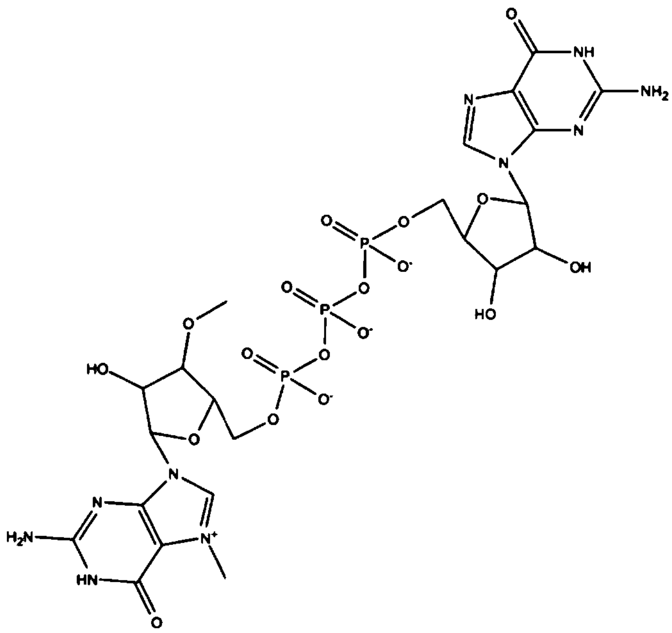

Formula 1

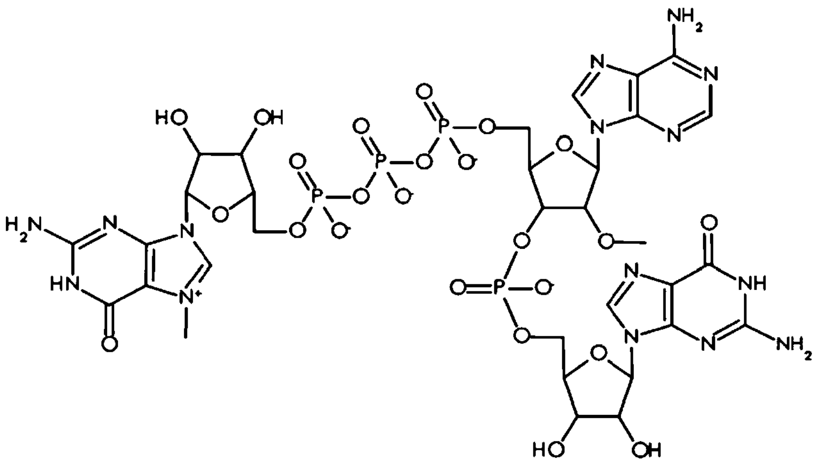

Formula 2

The ARCA cap analogue is a modified cap analogue in which the 3'-OH group proximal to m7G is substituted with -OCH3, and as a result of this substitution, RNA polymerase can only initiate transcription using the remaining hydroxyl groups, forcing the ARCA cap to be incorporated in a forward direction. The third-generation cap analogues, such as CleanCap AG, enable the formation of Cap 1 structures, with a significant increase in the capping rate compared to the second-generation cap analogues. However, this requires replacing the wild-type promoter from the original 5'-TAATA CGACTCACTATAGG-3' to 5'-TAATACGACTCACTATAAG-3'. Whether the promoter replacement leads to safety concerns or generates new impurities remains to be demonstrated. If the wild-type promoter is used, the capping rate remains low, and uncapped RNA products not only lead to waste of raw materials but also need to be removed later by column purification, increasing production costs. Therefore, if T7 RNA polymerase is modified, the utilization rate of cap analogues can be increased without the need for wild-type promoter replacement, which is of important significance for economical mRNA production and drug safety.

## Summary of the Application

In a first aspect, the present application provides a class of RNA polymerase variants, wherein the amino acid sequence of the variant comprises at least one mutation at amino acid positions selected from R386, R34, K172, Y178, D388, Q435, N437 or D438, relative to SEQ ID NO: 1, and the type of the mutation is selected from a substitution or a deletion.

In a second aspect, the present application provides one or more biological materials selected from:

1) a polynucleotide molecule encoding an RNA polymerase variant;
2) an expression vector comprising the polynucleotide molecule as described in 1); and
3) a host cell comprising the polynucleotide molecule as described in 1), or a host cell comprising the expression vector as described in 2).

In a third aspect, the present application provides a method for preparing the RNA polymerase variants described above.

In a fourth aspect, the present application provides a composition comprising at least one of the RNA polymerase variants as described in the present application.

In a fifth aspect, the present application provides a kit comprising at least one of the RNA polymerase variants as described in the present application.

In a sixth aspect, the present application further provides the use of the RNA polymerase variants, the composition or the kit described above in in-vitro transcription.

In a seventh aspect, the present application further provides a method for preparing RNA or capped RNA.

## Brief Description of the Drawings

FIG. 1 shows a schematic diagram of a double-stranded DNA template.

FIG. 2 shows complementary pairing of a cap analogue with a DNA template.
FIG. 3 shows a schematic diagram of the construction of a recombinant plasmid.
FIG. 4 shows the impact of an RNA polymerase and variants thereof (R386W, Q435A, Q435H, Q435T, N437F, N437T, D438T, D438L, D438I, D438P, D438V, R386A+R34A, R386M+R34A, R386M+Y178H, R386W+N437T, R386I+Del172 and R386F+Y178D) on the capping rate of mRNA.
FIG. 5 shows the impact of an RNA polymerase and variants thereof (WT, R386C, R386W, D388K, R386C+D438P, R386W+N437T, D388K+D438P, N437T and D438P) on the capping rate of mRNA.

## Detailed Description of Embodiments

RNA Polymerase Variants

The RNA polymerase variants provided in the present application are bacteriophage T7 RNA polymerase (T7 RNAP) variants. Amino acid sequences of the variants comprise, relative to SEQ ID NO: 1, at least one mutation at amino acid positions selected from R386, R34, K172, Y178, D388, Q435, N437 or D438, and the type of the mutation is selected from a substitution or a deletion.
In some embodiments, the substitution at position R34 of the variant is A.
In some embodiments, the type of the mutation at position K172 of the variant is a deletion.
In some embodiments, the substitution at position Y178 of the variant is selected from: H or D.
In some embodiments, the substitution at position R386 of the variant is selected from: C, W, M, A, I or F.
In some embodiments, the substitution at position D388 of the variant is K.
In some embodiments, the substitution at position Q435 of the variant is selected from: A, H or T.
In some embodiments, the substitution at position N437 of the variant is selected from: F or T.
In some embodiments, the substitution at position D438 of the variant is selected from: T, L, I, P or V.
In some embodiments, the amino acid sequences of the variants comprise, as compared to SEQ ID NO: 1, any of the following substitutions or groups of substitutions: R386W, R386C, D388K, Q435H, Q435T, Q435A, N437F, N437T, D438T, D438L, D438I, D438P, D438V, R386A+R34A, R386M+R34A, R386M+Y178H, R386W+N437T, R386I+del-K172, R386F+Y178D, R386C+D438P or D388K+D438P.
In some embodiments, the amino acid sequences of the RNA polymerase variants provided in the present application have at least 97%, at least 98%, at least 99% or higher sequence identity to a sequence as set forth in any one of SEQ ID NOs: 2-22. In some embodiments, the amino acid sequences of the variants are as set forth in any one of SEQ ID NOs: 2-22.

Polynucleotide Molecule

The polynucleotide molecule provided in the present application encodes any one of the RNA polymerase variants of the present application. In some embodiments, the polynucleotide molecule is as set forth in any one of SEQ ID NOs: 23-44.
The polynucleotide molecule of the present application may have various modifications in the coding region, provided that the amino acid sequences of the variants of the present application do not vary with the degeneracy of codons or with the preferred codons in the organism expressing the variant.

Expression Vector

The expression vector provided in the present application refers to a linear or circular DNA molecule, usually containing elements such as multiple cloning sites, resistance genes and origins of replication. In some embodiments, the expression vector of the present application is pQE-80L.
In some embodiments, the vector of the present application comprises a polynucleotide molecule encoding the RNA polymerase variant of the present application. In some embodiments, further, the vector further comprises one or more regulatory sequences (such as enhancer, promoter and terminator sequences) operably linked to the polynucleotide molecule encoding the variant.

Host Cell

The host cell provided in the present application is any cell that is advantageous for expression of the variant of the present application, i.e., any cell that is susceptible to transformation, transfection or transduction with the expression vector of the present application, including any progeny of a cell that differs from the parental cell due to mutations that occur during replication.

In some embodiments, the host cell of the present application comprises the polynucleotide molecule described above or the expression vector.

In some embodiments, the host cell is a prokaryotic cell, which is selected from a Gram-positive bacterium or a Gram-negative bacterium. In some embodiments, the host cell is a Gram-positive bacterium, including but not limited to: Bacillus, Clostridium, Enterococcus, Geobacillus, Lactobacillus, Lactococcus, Oceanobacillus, Staphylococcus, Streptococcus and Streptomyces. In some embodiments, the host cell is a Gram-negative bacterium, including but not limited to: Campylobacter, Escherichia coli, Flavobacterium, Fusobacterium, Helicobacter, Ilyobacter, Neisseria, Pseudomonas, Salmonella and Ureaplasma.

Method for Preparing Variants

The present application provides a method for preparing the RNA polymerase variant described above, the method comprising: (1) culturing the host cell of the present application under conditions suitable for expression of the variant; and (2) recovering the variant.

In some embodiments, the method for recovering the variant is a method well known in the art, such as centrifugation, filtration, treatment with a crystalline protein precipitant (salting out), extraction, sonication, ultrafiltration, dialysis, various chromatography methods such as molecular sieve chromatography (gel filtration), adsorption chromatography, ion exchange chromatography and affinity chromatography, HPLC, and combinations of the foregoing.

In some embodiments, the preparation method further comprises a step of purifying the variant, wherein the purification step is performed using a method well known in the art, such as chromatography (e.g., ion exchange chromatography, affinity chromatography or hydrophobic interaction chromatography) and ammonium sulphate precipitation.

Composition

The composition provided in the present application comprises at least one of the RNA polymerase variants of the present application.

The composition of the present application is a composition for storing the RNA polymerase variant. In some embodiments, the composition of the present application can optionally comprise, in addition to the RNA polymerase variant described above: a buffer component (such as Tris base, Tris-HCl, HEPES and MOPS), a salt (such as NaCl), an enzyme inhibitor (such as EDTA), a reducing agent (such as DTT), a surfactant (such as Triton X-100), a stabilizer (such as glycerine) and other components. In some embodiments, the composition for storing the RNA polymerase variant of the present application comprises: an RNA polymerase variant, Tris-HCl, NaCl, EDTA, DTT, Triton X-100 and glycerol.

The composition of the present application is an in-vitro transcription reaction composition. In some embodiments, the composition comprises, in addition to the RNA polymerase variant described above, one or more in-vitro transcription reaction reagents (such as a buffer component, a modified or unmodified nucleoside triphosphate, an RNase inhibitor, pyrophosphatase, a magnesium ion and water). In some embodiments, the composition further comprises a DNA template. In some embodiments, the composition further comprises a cap analogue.

In some embodiments, the in-vitro transcription reaction composition of the present application comprises: an RNA polymerase variant, a buffer component, a modified or unmodified nucleoside triphosphate, an RNase inhibitor, pyrophosphatase, a magnesium ion, water and a cap analogue. In some embodiments, the in-vitro transcription reaction composition of the present application comprises: an RNA polymerase variant, a buffer component, a modified or unmodified nucleoside triphosphate, an RNase inhibitor, pyrophosphatase, a magnesium ion, water, a cap analogue and a DNA template.

Kit

The kit provided in the present application comprises at least one of the RNA polymerase variants of the present application.

In some embodiments, the kit further comprise one or more in-vitro transcription reaction reagents (such as a buffer component, a modified or unmodified nucleoside triphosphate, an RNase inhibitor, pyrophosphatase, a magnesium ion and water). In some embodiments, the kit further comprises a cap analogue. In some embodiments, each component of the kit (if applicable) is provided in a liquid form (e.g., in a solution) or in a solid form (e.g., as a dry powder).

The kit of the present application comprises one or more containers containing one or more of the components of the present application and, optionally, instructions for use.

Application or Use

The present application provides the use of the RNA polymerase variants, the composition or the kit described above in in-vitro transcription.

The present application further provides the use of the RNA polymerase variants, the composition or the kit described above in a variety of methods including, but not limited to, preparation of RNA, preparation of RNA probes, preparation of RNA vaccines, preparation of proteins, etc.

Method for preparing RNA

The present application provides a method for preparing RNA. In some embodiments, the method comprises contacting a DNA template and a modified or unmodified nucleoside triphosphate with at least one of the RNA polymerase variants of the present application, and performing an incubation in an in-vitro transcription reaction system to obtain an RNA product of interest. In some embodiments, the RNA product can be dsRNA, ssRNA, mRNA, siRNA, miRNA, piRNA, shRNA or gRNA.

The present application further provides a method for preparing capped mRNA. In some embodiments, the method comprises contacting a DNA template, a modified or unmodified nucleoside triphosphate and a cap analogue with at least one of the RNA polymerase variants of the present application, and performing an incubation in an in-vitro transcription reaction system to obtain a product of interest.

In-vitro transcription reaction systems and incubation conditions suitable for the production of RNA products or capped mRNA products are well known in the art. Those of ordinary skill in the art can determine appropriate pH value of the reaction system, reaction temperature, reaction time, salt concentration, or whether to add exogenous cofactors, etc., while taking into account the optimal activity of the RNA polymerase. In some embodiments, the in-vitro transcription reaction system of the present application comprises in-vitro transcription reaction reagents including: one or more buffer components, a modified or unmodified nucleoside triphosphate, an RNase inhibitor, pyrophosphatase, a magnesium ion, water, etc. In some embodiments, in the incubation step of the present application, the incubation temperature is 30°C-50°C, preferably 37°C. In some embodiments, in the incubation step of the present application, the incubation time is 20-240 min, preferably 60 min.

In some embodiments, as compared to using the wild-type RNA polymerase, preparing the RNA product or capped mRNA product by using the method of the present application achieves a higher yield, and/or higher integrity, and/or a lower content of dsRNA impurities, and/or more capped mRNA products, etc.

In some embodiments, as compared to using the wild-type RNA polymerase, preparing the capped mRNA product by using the method of the present application can lead to an increase in the utilization rate of cap analogues, wherein the capping rate of the obtained mRNA product can be increased to at least 91%, at least 92%, at least 93%, at least 94%, at least 95%, at least 96%, at least 97%, at least 98%, at least 99% or 100%.

Cap Analogue

The cap analogue used in the method for preparing a capped mRNA product or the cap analogue in the in-vitro transcription reaction composition of the present application refers to a molecule that can be complementary to a nucleotide molecule on a DNA template at the transcription start site.

In some embodiments, the cap analogue is selected from a dinucleotide cap, a trinucleotide cap or a tetranucleotide cap. In some embodiments, the cap analogue is a trinucleotide cap, which is selected from GAA, GAC, GAG, GAU, GCA, GCC, GCG, GCU, GGA, GGC, GGG, GGU, GUA, GUC, GUG and GUU. In some embodiments, the trinucleotide cap is selected from m7GpppApA, m7GpppApC, m7GpppApG, m7GpppApU, m7GpppCpA, m7GpppCpC, m7GpppCpG, m7GpppCpU, m7GpppGpA, m7GpppGpC, m7GpppGpG, m7GpppGpU, m7GpppUpA, m7GpppUpC, m7GpppUpG and m7GpppUpU. In some embodiments, the trinucleotide cap is selected from m7G3'OMepppApA, m7G3'OMepppApC, m7G3'OMepppApG, m7G3'OMepppApU, m7G3'OMepppCpA, m7G3'OMepppCpC, m7G3'OMepppCpG, m7G3'OMepppCpU, m7G3'OMepppGpA, m7G3'OMepppGpC, m7G3'OMepppGpG, m7G3'OMepppGpU, m7G3'OMepppUpA, m7G3'OMepppUpC, m7G3'OMepppUpG and m7G3'OMepppUpU. In some embodiments, the trinucleotide cap cis selected from m7G3'OMepppA2'OMepA, m7G3'OMepppA2'OMepC, m7G3'OMepppA2'OMepG, m7G3'OMepppA2'OMepU, m7G3'OMepppC2'OMepA, m7G3'OMepppC2'OMepC, m7G3'OMepppC2'OMepG, m7G3'OMepppC2'OMepU, m7G3'OMepppG2'OMepA, m7G3'OMepppG2'OMepC, m7G3'OMepppG2'OMepG, m7G3'OMepppG2'OMepU, m7G3'OMepppU2'OMepA, m7G3'OMepppU2'OMepC, m7G3'OMepppU2'OMepG and m7G3'OMepppU2'OMepU. In some embodiments, the trinucleotide cap is selected from m7GpppA2'OMepA, m7GpppA2'OMepC, m7GpppA2'OMepG, m7GpppA2'OMepU, m7GpppC2'OMepA, m7GpppC2'OMepC, m7GpppC2'OMepG, m7GpppC2'OMepU, m7GpppG2'OMepA, m7GpppG2'OMepC, m7GpppG2'OMepG, m7GpppG2'OMepU, m7GpppU2'OMepA, m7GpppU2'OMepC, m7GpppU2'OMepG and m7GpppU2'OMepU.

In some embodiments, the cap analogue of the present application is preferably m7GpppA2'OMepG.

In some embodiments, when the capped mRNA product is prepared using a trinucleotide cap GAG (such as m7GpppA2'OMepG), or in an in-vitro transcription reaction composition containing the cap analogue, the first nucleotide at position +1 of the DNA template molecule (sense strand) is G, and the second nucleotide at position +2 is G. In some embodiments, the nucleotide residue in the m7GpppA2'OMepG cap analogue is capable of complementary pairing with position +1 of the antisense strand of the DNA template molecule (see FIG. 2).

In-Vitro Transcription Reaction Reagent

The in-vitro transcription reaction reagent of the present application comprises a buffer component, a nucleoside triphosphate, an RNase inhibitor, an inorganic pyrophosphatase, a magnesium ion, water (such as DEPC-water, RNase-free water, DNase-free water, sterilized purified water, deionized water and distilled water), etc.

In some embodiments, the buffer component is selected from one or more of a phosphate buffer, a Tris buffer, an MOPS buffer, an HEPES buffer, a citrate buffer, an acetate buffer, a malate buffer, an MES buffer, a histidine buffer, a PIPES buffer, a bis-tris buffer and an ethanolamine buffer.

In some embodiments, the nucleoside triphosphate is selected from a modified or unmodified nucleoside triphosphate (including an analogue thereof). In some embodiments, the nucleoside triphosphate is selected from unmodified ATP, GTP, CTP or UTP. In some embodiments, the nucleoside triphosphate is selected from a modified nucleoside triphosphate, wherein the type of modifications on the nucleoside includes, but is not limited to, m1A (N1-methyladenosine), m6A (N6-methyladenosine), m5C (5-methylcytidine), 5moU (5-methoxyuridine), $\psi$ (pseudouridine), mly (N1-methyl-pseudouridine), and a nucleoside triphosphate with a label (the label may be biotin, a fluorescent substance, digoxin, a radioactive element, etc.).

In some embodiments, the in-vitro transcription reaction reagent of the present application is selected from any commercially available in-vitro transcription reagent for RNA.

Other Embodiments:

1. An RNA polymerase variant, wherein the amino acid sequence of the variant comprises at least one substitution or deletion at amino acid positions selected from R386, R34, K172, Y178, D388, Q435, N437 or D438, relative to SEQ ID NO: 1.
2. The variant according to item 1, wherein:

   (1) the substitution at position R386 is selected from C or W;
   (2) the substitution at position D388 is K;
   (3) the substitution at position Q435 is selected from A, H or T;
   (4) the substitution at position N437 is selected from F or T;
   (5) the substitution at position D438 is selected from T, L, I, P or V;
   (6) the substitution at position R34 is selected from A;
   (7) the mutation at position K172 is a deletion; and
   (8) the substitution at position Y178 is selected from H or D.

3. The variant according to item 1, wherein the amino acid sequence of the variant comprises any one of mutation at positions selected from R386W, R386C, D388K, Q435A, Q435H, Q435T, N437F, N437T, D438T, D438L, D438I, D438P, D438V, R386A+R34A, R386M+R34A, R386M+Y178H, R386W+N437T, R386I+del-K172, R386F+Y178D, D388K+D438P or R386C+D438P, relative to SEQ ID NO: 1.
4. The variant according to item 1, wherein the amino acid sequence of the variant is set forth in any one of SEQ ID NOs: 2-22.
5. A polynucleotide molecule encoding the variant according to any one of items 1-4.
6. An expression vector comprising the polynucleotide molecule according to item 5.
7. A host cell comprising the polynucleotide molecule according to item 5 or the expression vector according to item 6.
8. A method for preparing the variant according to any one of items 1-4, comprising:

   (1) culturing the host cell according to item 7; and
   (2) recovering the variant.

9. A composition comprising the variant according to any one of items 1-4.

10. The composition according to item 9, further comprising a cap analogue.
11. The composition according to item 9 or 10, further comprising a DNA template.
12. A kit comprising the variant according to any one of items 1-4.
13. Use of the variant according to any one of items 1-4, the composition according to any one of items 9-11, or the kit according to item 12 in in-vitro transcription.
14. A method for preparing RNA, comprising contacting a DNA template and a modified or unmodified nucleoside triphosphate with the RNA polymerase variant according to any one of items 1-4, and performing an incubation in an in-vitro transcription reaction system to obtain an RNA product of interest.
15. A method for preparing capped mRNA, comprising contacting a DNA template, a modified or unmodified nucleoside triphosphate and a cap analogue with the RNA polymerase variant according to any one of items 1-4, and performing an incubation in an in-vitro transcription reaction system to obtain a product of interest.
16. The method according to item 15, wherein the cap analogue is a trinucleotide cap, preferably m7GpppA2'OMepG.
17. The composition according to item 11 or the method according to any one of items 14-16, wherein the DNA template has 2'-deoxyguanosine residues at positions + 1 and + 2.
18. Use of the variant according to any one of items 1-4, the composition according to any one of items 9-11, or the kit according to item 12 in transcription of a DNA template having 2'-deoxyguanosine residues at positions + 1 and + 2 of the template.

Beneficial Effects

The present application provides a class of RNA polymerase variants and a preparation method therefor. By modifying the wild-type T7 RNA polymerase, RNA polymerase variants with relatively high catalytic efficiency are obtained. By using such variants, the capping rate of mRNA products during in-vitro transcription can be increased, thereby obtaining more capped mRNA products. In addition, the present application further provides a method for preparing capped mRNA. By using the method, the yield of capped mRNA products can be increased, and the amount of cap analogues used can be reduced at the same time, which avoids waste of raw materials, saves production costs, and is of great significance for the economical production of RNA.

In the examples of the present application, one unit of enzyme activity (U) is defined as the amount of enzyme required to incorporate 1 nmol [3H] ATP into an acid-insoluble precipitate within one hour under conditions of 37°C and pH 8.0.

## Example 1 Preparation of RNA Polymerase Variants

RNA polymerases shown in Table 1 were subjected to PCR amplification after DNA sequence synthesis (SEQ ID NOs: 23-44), and then introduced into the BseRI and HindIII cleavage sites of the expression vector pQE-80L to obtain recombinant expression vectors. The constructed vectors were introduced into E. coli BL21 (DE3) by transformation techniques, plated in an ampicillin resistant LB plate, and then placed in an incubator at 37°C overnight. The grown single colonies were subjected to plasmid extraction and sequencing. Finally, recombinant engineered bacteria containing the gene of interest were obtained. The successfully sequenced E. coli recombinant strains were inoculated into an LB medium for overnight activation culture, and then inoculated into a fermentation broth (an LB medium) at 1%-5% V/V. When the OD 600 value reached 0.6-0.8, IPTG at a final concentration of 0.5 mol/L was added, followed by an additional incubation for 4-6 h. The strains were collected by centrifugation at 12,000 rpm at 4°C. The collected strains were washed with 0.2 M PBS buffer at pH 7.0, and cell pellets were obtained. After lysis by sonication, affinity chromatography purification was performed to obtain the stock solution of RNA polymerase.

The correspondence of RNA polymerase variants to amino acid sequences is shown in Table 1.

Table 1:

| RNA polymerase and variants thereof | Amino acid sequence |
|---|---|
| WT (SEQ ID NO: 1) | MNTINIAKNDFSDIELAAIPFNTLADHYGERLAREQLALEHESYEMGEARFRKMFERQLKAGE<br>VADNAAAKPLITTLLPKMIARINDWFEEVKAKRGKRPTAFQFLQEIKPEAVAYITIKTTLACLTS<br>ADNTTVQAVASAIGRAIEDEARFGRIRDLEAKHFKKNVEEQLNKRVGHVYKKAFMQVVEAD<br>MLSKGLLGGEAWSSWHKEDSIHVGVRCIEMLIESTGMVSLHRQNAGVVGQDSETIELAPEYA<br>EAIATRAGALAGISPMFQPCVVPPKPWTGITGGGYWANGRRPLALVRTHSKKALMRYEDVYM<br>PEVYKAINIAQNTAWKINKKVLAVANVITKWKHCPVEDIPAIEREELPMKPEDIDMNPEALTAW<br>KRAAAAVYRKDKARKSRRISLEFMLEQANKFANHKAIWFPYNMDWRGRVYAVSMFNPQGN<br>DMTKGLLTLAKGKPIGKEGYYWLKIHGANCAGVDKVPFPERIKFIEENHENIMACAKSPLENT<br>WWAEQDSPFCFLAFCFEYAGVQHHGLSYNCSLPLAFDGSCSGIQHFSAMLRDEVGGRAVNLL<br>PSETVQDIYGIVAKKVNEILQADAINGTDNEVVTVTDENTGEISEKVKLGTKALAGQWLAYG<br>VTRSVTKRSVMTLAYGSKEFGFRQQVLEDTIQPAIDSGKGLMFTQPNQAAGYMAKLIWESVS<br>VTVVAAVEAMNWLKSAAKLLAAEVKDKKTGEILRKRCAVHWVTPDGFPVWQEYKKPIQTR<br>LNLMFLGQFRLQPTINTNKDSEIDAHKQESGIAPNFVHSQDGSHLRKTVVWAHEKYGIESFALI<br>HDSFGTIPADAANLFKAVRETMVDTYESCDVLADFYDQFADQLHESQLDKMPALPAKGNLNL<br>RDILESDFAFA |

(continued)

| RNA polymerase and variants thereof | Amino acid sequence |
|---|---|
| R386C (SEQ ID NO: 2) | MNTINIAKNDFSDIELAAIPFNTLADHYGERLAREQLALEHESYEMGEARFRKMFERQLKAGE<br>VADNAAAKPLITTLLPKMIARINDWFEEVKAKRGKRPTAFQFLQEIKPEAVAYITIKTTLACLTS<br>ADNTTVQAVASAIGRAIEDEARFGRIRDLEAKHFKKNVEEQLNKRVGHVYKKAFMQVVEAD<br>MLSKGLLGGEAWSSWHKEDSIHVGVRCIEMLIESTGMVSLHRQNAGVVGQDSETIELAPEYA<br>EAIATRAGALAGISPMFQPCVVPPKPWTGITGGGYWANGRRPLALVRTHSKKALMRYEDVYM<br>PEVYKAINIAQNTAWKINKKVLAVANVITKWKHCPVEDIPAIEREELPMKPEDIDMNPEALTAW<br>KRAAAAVYCKDKARKSRRISLEFMLEQANKFANHKAIWFPYNMDWRGRVYAVSMFNPQGN<br>DMTKGLLTLAKGKPIGKEGYYWLKIHGANCAGVDKVPFPERIKFIEENHENIMACAKSPLENT<br>WWAEQDSPFCFLAFCFEYAGVQHHGLSYNCSLPLAFDGSCSGIQHFSAMLRDEVGGRAVNLL<br>PSETVQDIYGIVAKKVNEILQADAINGTDNEVVTVTDENTGEISEKVKLGTKALAGQWLAYG<br>VTRSVTKRSVMTLAYGSKEFGFRQQVLEDTIQPAIDSGKGLMFTQPNQAAGYMAKLIWESVS<br>VTVVAAVEAMNWLKSAAKLLAAEVKDKKTGEILRKRCAVHWVTPDGFPVWQEYKKPIQTR<br>LNLMFLGQFRLQPTINTNKDSEIDAHKQESGIAPNFVHSQDGSHLRKTVVWAHEKYGIESFALI<br>HDSFGTIPADAANLFKAVRETMVDTYESCDVLADFYDQFADQLHESQLDKMPALPAKGNLNL<br>RDILESDFAFA |

(continued)

| RNA polymerase and variants thereof | Amino acid sequence |
| --- | --- |
| R386W (SEQ ID NO: 3) | MNTINIAKNDFSDIELAAIPFNTLADHYGERLAREQLALEHESYEMGEARFRKMFERQLKAGE<br>VADNAAAKPLITTLLPKMIARINDWFEEVKAKRGKRPTAFQFLQEIKPEAVAYITIKTTLACLTS<br>ADNTTVQAVASAIGRAIEDEARFGRIRDLEAKHFKKNVEEQLNKRVGHVYKKAFMQVVEAD<br>MLSKGLLGGEAWSSWHKEDSIHVGVRCIEMLIESTGMVSLHRQNAGVVGQDSETIELAPEYA<br>EAIATRAGALAGISPMFQPCVVPPKPWTGITGGGYWANGRRPLALVRTHSKKALMRYEDVYM<br>PEVYKAINIAQNTAWKINKKVLAVANVITKWKHCPVEDIPAIEREELPMKPEDIDMNPEALTAW<br>KRAAAAVYWKDKARKSRRISLEFMLEQANKFANHKAIWFPYNMDWRGRVYAVSMFNPQGN<br>DMTKGLLTLAKGKPIGKEGYYWLKIHGANCAGVDKVPFPERIKFIEENHENIMACAKSPLENT<br>WWAEQDSPFCFLAFCFEYAGVQHHGLSYNCSLPLAFDGSCSGIQHFSAMLRDEVGGRAVNLL<br>PSETVQDIYGIVAKKVNEILQADAINGTDNEVVTVTDENTGEISEKVKLGTKALAGQWLAYG<br>VTRSVTKRSVMTLAYGSKEFGFRQQVLEDTIQPAIDSGKGLMFTQPNQAAGYMAKLIWESVS<br>VTVVAAVEAMNWLKSAAKLLAAEVKDKKTGEILRKRCAVHWVTPDGFPVWQEYKKPIQTR<br>LNLMFLGQFRLQPTINTNKDSEIDAHKQESGIAPNFVHSQDGSHLRKTVVWAHEKYGIESFALI<br>HDSFGTIPADAANLFKAVRETMVDTYESCDVLADFYDQFADQLHESQLDKMPALPAKGNLNL<br>RDILESDFAFA |

(continued)

| RNA polymerase and variants thereof | Amino acid sequence |
|---|---|
| D388K (SEQ ID NO: 4) | MNTINIAKNDFSDIELAAIPFNTLADHYGERLAREQLALEHESYEMGEARFRKMFERQLKAGE<br>VADNAAAKPLITTLLPKMIARINDWFEEVKAKRGKRPTAFQFLQEIKPEAVAYITIKTTLACLTS<br>ADNTTVQAVASAIGRAIEDEARFGRIRDLEAKHFKKNVEEQLNKRVGHVYKKAFMQVVEAD<br>MLSKGLLGGEAWSSWHKEDSIHVGVRCIEMLIESTGMVSLHRQNAGVVGQDSETIELAPEYA<br>EAIATRAGALAGISPMFQPCVVPPKPWTGITGGGYWANGRRPLALVRTHSKKALMRYEDVYM<br>PEVYKAINIAQNTAWKINKKVLAVANVITKWKHCPVEDIPAIEREELPMKPEDIDMNPEALTAW<br>KRAAAAVYRKKKARKSRRISLEFMLEQANKFANHKAIWFPYNMDWRGRVYAVSMFNPQGN<br>DMTKGLLTLAKGKPIGKEGYYWLKIHGANCAGVDKVPFPERIKFIEENHENIMACAKSPLENT<br>WWAEQDSPFCFLAFCFEYAGVQHHGLSYNCSLPLAFDGSCSGIQHFSAMLRDEVGGRAVNLL<br>PSETVQDIYGIVAKKVNEILQADAINGTDNEVVTVTDENTGEISEKVKLGTKALAGQWLAYG<br>VTRSVTKRSVMTLAYGSKEFGFRQQVLEDTIQPAIDSGKGLMFTQPNQAAGYMAKLIWESVS<br>VTVVAAVEAMNWLKSAAKLLAAEVKDKKTGEILRKRCAVHWVTPDGFPVWQEYKKPIQTR<br>LNLMFLGQFRLQPTINTNKDSEIDAHKQESGIAPNFVHSQDGSHLRKTVVWAHEKYGIESFALI<br>HDSFGTIPADAANLFKAVRETMVDTYESCDVLADFYDQFADQLHESQLDKMPALPAKGNLNL<br>RDILESDFAFA |

(continued)

| RNA polymerase and variants thereof | Amino acid sequence |
| --- | --- |
| Q435A (SEQ ID NO: 5) | MNTINIAKNDFSDIELAAIPFNTLADHYGERLAREQLALEHESYEMGEARFRKMFERQLKAGE<br>VADNAAAKPLITTLLPKMIARINDWFEEVKAKRGKRPTAFQFLQEIKPEAVAYITIKTTLACLTS<br>ADNTTVQAVASAIGRAIEDEARFGRIRDLEAKHFKKNVEEQLNKRVGHVYKKAFMQVVEAD<br>MLSKGLLGGEAWSSWHKEDSIHVGVRCIEMLIESTGMVSLHRQNAGVVGQDSETIELAPEYA<br>EAIATRAGALAGISPMFQPCVVPPKPWTGITGGGYWANGRRPLALVRTHSKKALMRYEDVYM<br>PEVYKAINIAQNTAWKINKKVLAVANVITKWKHCPVEDIPAIEREELPMKPEDIDMNPEALTAW<br>KRAAAAVYRKDKARKSRRISLEFMLEQANKFANHKAIWFPYNMDWRGRVYAVSMFNPAGN<br>DMTKGLLTLAKGKPIGKEGYYWLKIHGANCAGVDKVPFPERIKFIEENHENIMACAKSPLENT<br>WWAEQDSPFCFLAFCFEYAGVQHHGLSYNCSLPLAFDGSCSGIQHFSAMLRDEVGGRAVNLL<br>PSETVQDIYGIVAKKVNEILQADAINGTDNEVVTVTDENTGEISEKVKLGTKALAGQWLAYG<br>VTRSVTKRSVMTLAYGSKEFGFRQQVLEDTIQPAIDSGKGLMFTQPNQAAGYMAKLIWESVS<br>VTVVAAVEAMNWLKSAAKLLAAEVKDKKTGEILRKRCAVHWVTPDGFPVWQEYKKPIQTR<br>LNLMFLGQFRLQPTINTNKDSEIDAHKQESGIAPNFVHSQDGSHLRKTVVWAHEKYGIESFALI<br>HDSFGTIPADAANLFKAVRETMVDTYESCDVLADFYDQFADQLHESQLDKMPALPAKGNLNL<br>RDILESDFAFA |

(continued)

| RNA polymerase and variants thereof | Amino acid sequence |
|---|---|
| Q435H (SEQ ID NO: 6) | MNTINIAKNDFSDIELAAIPFNTLADHYGERLAREQLALEHESYEMGEARFRKMFERQLKAGE<br>VADNAAAKPLITTLLPKMIARINDWFEEVKAKRGKRPTAFQFLQEIKPEAVAYITIKTTLACLTS<br>ADNTTVQAVASAIGRAIEDEARFGRIRDLEAKHFKKNVEEQLNKRVGHVYKKAFMQVVEAD<br>MLSKGLLGGEAWSSWHKEDSIHVGVRCIEMLIESTGMVSLHRQNAGVVGQDSETIELAPEYA<br>EAIATRAGALAGISPMFQPCVVPPKPWTGITGGGYWANGRRPLALVRTHSKKALMRYEDVYM<br>PEVYKAINIAQNTAWKINKKVLAVANVITKWKHCPVEDIPAIEREELPMKPEDIDMNPEALTAW<br>KRAAAAVYRKDKARKSRRISLEFMLEQANKFANHKAIWFPYNMDWRGRVYAVSMFNPHGN<br>DMTKGLLTLAKGKPIGKEGYYWLKIHGANCAGVDKVPFPERIKFIEENHENIMACAKSPLENT<br>WWAEQDSPFCFLAFCFEYAGVQHHGLSYNCSLPLAFDGSCSGIQHFSAMLRDEVGGRAVNLL<br>PSETVQDIYGIVAKKVNEILQADAINGTDNEVVTVTDENTGEISEKVKLGTKALAGQWLAYG<br>VTRSVTKRSVMTLAYGSKEFGFRQQVLEDTIQPAIDSGKGLMFTQPNQAAGYMAKLIWESVS<br>VTVVAAVEAMNWLKSAAKLLAAEVKDKKTGEILRKRCAVHWVTPDGFPVWQEYKKPIQTR<br>LNLMFLGQFRLQPTINTNKDSEIDAHKQESGIAPNFVHSQDGSHLRKTVVWAHEKYGIESFALI<br>HDSFGTIPADAANLFKAVRETMVDTYESCDVLADFYDQFADQLHESQLDKMPALPAKGNLNL<br>RDILESDFAFA |

(continued)

| RNA polymerase and variants thereof | Amino acid sequence |
|---|---|
| Q435T (SEQ ID NO: 7) | MNTINIAKNDFSDIELAAIPFNTLADHYGERLAREQLALEHESYEMGEARFRKMFERQLKAGE VADNAAAKPLITTLLPKMIARINDWFEEVKAKRGKRPTAFQFLQEIKPEAVAYITIKTTLACLTS ADNTTVQAVASAIGRAIEDEARFGRIRDLEAKHFKKNVEEQLNKRVGHVYKKAFMQVVEAD MLSKGLLGGEAWSSWHKEDSIHVGVRCIEMLIESTGMVSLHRQNAGVVGQDSETIELAPEYA EAIATRAGALAGISPMFQPCVVPPKPWTGITGGGYWANGRRPLALVRTHSKKALMRYEDVYM PEVYKAINIAQNTAWKINKKVLAVANVITKWKHCPVEDIPAIEREELPMKPEDIDMNPEALTAW KRAAAAVYRKDKARKSRRISLEFMLEQANKFANHKAIWFPYNMDWRGRVYAVSMFNPTGN DMTKGLLTLAKGKPIGKEGYYWLKIHGANCAGVDKVPFPERIKFIEENHENIMACAKSPLENT WWAEQDSPFCFLAFCFEYAGVQHHGLSYNCSLPLAFDGSCSGIQHFSAMLRDEVGGRAVNLL PSETVQDIYGIVAKKVNEILQADAINGTDNEVVTVTDENTGEISEKVKLGTKALAGQWLAYG VTRSVTKRSVMTLAYGSKEFGFRQQVLEDTIQPAIDSGKGLMFTQPNQAAGYMAKLIWESVS VTVVAAVEAMNWLKSAAKLLAAEVKDKKTGEILRKRCAVHWVTPDGFPVWQEYKKPIQTR LNLMFLGQFRLQPTINTNKDSEIDAHKQESGIAPNFVHSQDGSHLRKTVVWAHEKYGIESFALI HDSFGTIPADAANLFKAVRETMVDTYESCDVLADFYDQFADQLHESQLDKMPALPAKGNLNL RDILESDFAFA |

(continued)

| RNA polymerase and variants thereof | Amino acid sequence |
|---|---|
| N437F (SEQ ID NO: 8) | MNTINIAKNDFSDIELAAIPFNTLADHYGERLAREQLALEHESYEMGEARFRKMFERQLKAGE<br>VADNAAAKPLITTLLPKMIARINDWFEEVKAKRGKRPTAFQFLQEIKPEAVAYITIKTTLACLTS<br>ADNTTVQAVASAIGRAIEDEARFGRIRDLEAKHFKKNVEEQLNKRVGHVYKKAFMQVVEAD<br>MLSKGLLGGEAWSSWHKEDSIHVGVRCIEMLIESTGMVSLHRQNAGVVGQDSETIELAPEYA<br>EAIATRAGALAGISPMFQPCVVPPKPWTGITGGGYWANGRRPLALVRTHSKKALMRYEDVYM<br>PEVYKAINIAQNTAWKINKKVLAVANVITKWKHCPVEDIPAIEREELPMKPEDIDMNPEALTAW<br>KRAAAAVYRKDKARKSRRISLEFMLEQANKFANHKAIWFPYNMDWRGRVYAVSMFNPQGFD<br>MTKGLLTLAKGKPIGKEGYYWLKIHGANCAGVDKVPFPERIKFIEENHENIMACAKSPLENT<br>WWAEQDSPFCFLAFCFEYAGVQHHGLSYNCSLPLAFDGSCSGIQHFSAMLRDEVGGRAVNLL<br>PSETVQDIYGIVAKKVNEILQADAINGTDNEVVTVTDENTGEISEKVKLGTKALAGQWLAYG<br>VTRSVTKRSVMTLAYGSKEFGFRQQVLEDTIQPAIDSGKGLMFTQPNQAAGYMAKLIWESVS<br>VTVVAAVEAMNWLKSAAKLLAAEVKDKKTGEILRKRCAVHWVTPDGFPVWQEYKKPIQTR<br>LNLMFLGQFRLQPTINTNKDSEIDAHKQESGIAPNFVHSQDGSHLRKTVVWAHEKYGIESFALI<br>HDSFGTIPADAANLFKAVRETMVDTYESCDVLADFYDQFADQLHESQLDKMPALPAKGNLNL<br>RDILESDFAFA |

(continued)

| RNA polymerase and variants thereof | Amino acid sequence |
|---|---|
| N437T (SEQ ID NO: 9) | MNTINIAKNDFSDIELAAIPFNTLADHYGERLAREQLALEHESYEMGEARFRKMFERQLKAGE VADNAAAKPLITTLLPKMIARINDWFEEVKAKRGKRPTAFQFLQEIKPEAVAYITIKTTLACLTS ADNTTVQAVASAIGRAIEDEARFGRIRDLEAKHFKKNVEEQLNKRVGHVYKKAFMQVVEAD MLSKGLLGGEAWSSWHKEDSIHVGVRCIEMLIESTGMVSLHRQNAGVVGQDSETIELAPEYA EAIATRAGALAGISPMFQPCVVPPKPWTGITGGGYWANGRRPLALVRTHSKKALMRYEDVYM PEVYKAINIAQNTAWKINKKVLAVANVITKWKHCPVEDIPAIEREELPMKPEDIDMNPEALTAW KRAAAAVYRKDKARKSRRISLEFMLEQANKFANHKAIWFPYNMDWRGRVYAVSMFNPQGT DMTKGLLTLAKGKPIGKEGYYWLKIHGANCAGVDKVPFPERIKFIEENHENIMACAKSPLENT WWAEQDSPFCFLAFCFEYAGVQHHGLSYNCSLPLAFDGSCSGIQHFSAMLRDEVGGRAVNLL PSETVQDIYGIVAKKVNEILQADAINGTDNEVVTVTDENTGEISEKVKLGTKALAGQWLAYG VTRSVTKRSVMTLAYGSKEFGFRQQVLEDTIQPAIDSGKGLMFTQPNQAAGYMAKLIWESVS VTVVAAVEAMNWLKSAAKLLAAEVKDKKTGEILRKRCAVHWVTPDGFPVWQEYKKPIQTR LNLMFLGQFRLQPTINTNKDSEIDAHKQESGIAPNFVHSQDGSHLRKTVVWAHEKYGIESFALI HDSFGTIPADAANLFKAVRETMVDTYESCDVLADFYDQFADQLHESQLDKMPALPAKGNLNL RDILESDFAFA |
| D438T | MNTINIAKNDFSDIELAAIPFNTLADHYGERLAREQLALEHESYEMGEARFRKMFERQLKAGE |

(continued)

| RNA polymerase and variants thereof | Amino acid sequence |
|---|---|
| (SEQ ID NO: 10) | VADNAAAKPLITTLLPKMIARINDWFEEVKAKRGKRPTAFQFLQEIKPEAVAYITIKTTLACLTS<br>ADNTTVQAVASAIGRAIEDEARFGRIRDLEAKHFKKNVEEQLNKRVGHVYKKAFMQVVEAD<br>MLSKGLLGGEAWSSWHKEDSIHVGVRCIEMLIESTGMVSLHRQNAGVVGQDSETIELAPEYA<br>EAIATRAGALAGISPMFQPCVVPPKPWTGITGGGYWANGRRPLALVRTHSKKALMRYEDVYM<br>PEVYKAINIAQNTAWKINKKVLAVANVITKWKHCPVEDIPAIEREELPMKPEDIDMNPEALTAW<br>KRAAAAVYRKDKARKSRRISLEFMLEQANKFANHKAIWFPYNMDWRGRVYAVSMFNPQGN<br>TMTKGLLTLAKGKPIGKEGYYWLKIHGANCAGVDKVPFPERIKFIEENHENIMACAKSPLENT<br>WWAEQDSPFCFLAFCFEYAGVQHHGLSYNCSLPLAFDGSCSGIQHFSAMLRDEVGGRAVNLL<br>PSETVQDIYGIVAKKVNEILQADAINGTDNEVVTVTDENTGEISEKVKLGTKALAGQWLAYG<br>VTRSVTKRSVMTLAYGSKEFGFRQQVLEDTIQPAIDSGKGLMFTQPNQAAGYMAKLIWESVS<br>VTVVAAVEAMNWLKSAAKLLAAEVKDKKTGEILRKRCAVHWVTPDGFPVWQEYKKPIQTR<br>LNLMFLGQFRLQPTINTNKDSEIDAHKQESGIAPNFVHSQDGSHLRKTVVWAHEKYGIESFALI<br>HDSFGTIPADAANLFKAVRETMVDTYESCDVLADFYDQFADQLHESQLDKMPALPAKGNLNL<br>RDILESDFAFA |

(continued)

| RNA polymerase and variants thereof | Amino acid sequence |
|---|---|
| D438L (SEQ ID NO: 11) | MNTINIAKNDFSDIELAAIPFNTLADHYGERLAREQLALEHESYEMGEARFRKMFERQLKAGE<br>VADNAAAKPLITTLLPKMIARINDWFEEVKAKRGKRPTAFQFLQEIKPEAVAYITIKTTLACLTS<br>ADNTTVQAVASAIGRAIEDEARFGRIRDLEAKHFKKNVEEQLNKRVGHVYKKAFMQVVEAD<br>MLSKGLLGGEAWSSWHKEDSIHVGVRCIEMLIESTGMVSLHRQNAGVVGQDSETIELAPEYA<br>EAIATRAGALAGISPMFQPCVVPPKPWTGITGGGYWANGRRPLALVRTHSKKALMRYEDVYM<br>PEVYKAINIAQNTAWKINKKVLAVANVITKWKHCPVEDIPAIEREELPMKPEDIDMNPEALTAW<br>KRAAAAVYRKDKARKSRRISLEFMLEQANKFANHKAIWFPYNMDWRGRVYAVSMFNPQGN<br>LMTKGLLTLAKGKPIGKEGYYWLKIHGANCAGVDKVPFPERIKFIEENHENIMACAKSPLENT<br>WWAEQDSPFCFLAFCFEYAGVQHHGLSYNCSLPLAFDGSCSGIQHFSAMLRDEVGGRAVNLL<br>PSETVQDIYGIVAKKVNEILQADAINGTDNEVVTVTDENTGEISEKVKLGTKALAGQWLAYG<br>VTRSVTKRSVMTLAYGSKEFGFRQQVLEDTIQPAIDSGKGLMFTQPNQAAGYMAKLIWESVS<br>VTVVAAVEAMNWLKSAAKLLAAEVKDKKTGEILRKRCAVHWVTPDGFPVWQEYKKPIQTR<br>LNLMFLGQFRLQPTINTNKDSEIDAHKQESGIAPNFVHSQDGSHLRKTVVWAHEKYGIESFALI<br>HDSFGTIPADAANLFKAVRETMVDTYESCDVLADFYDQFADQLHESQLDKMPALPAKGNLNL<br>RDILESDFAFA |

(continued)

| RNA polymerase and variants thereof | Amino acid sequence |
|---|---|
| D438I (SEQ ID NO: 12) | MNTINIAKNDFSDIELAAIPFNTLADHYGERLAREQLALEHESYEMGEARFRKMFERQLKAGE VADNAAAKPLITTLLPKMIARINDWFEEVKAKRGKRPTAFQFLQEIKPEAVAYITIKTTLACLTS ADNTTVQAVASAIGRAIEDEARFGRIRDLEAKHFKKNVEEQLNKRVGHVYKKAFMQVVEAD MLSKGLLGGEAWSSWHKEDSIHVGVRCIEMLIESTGMVSLHRQNAGVVGQDSETIELAPEYA EAIATRAGALAGISPMFQPCVVPPKPWTGITGGGYWANGRRPLALVRTHSKKALMRYEDVYM PEVYKAINIAQNTAWKINKKVLAVANVITKWKHCPVEDIPAIEREELPMKPEDIDMNPEALTAW KRAAAAVYRKDKARKSRRISLEFMLEQANKFANHKAIWFPYNMDWRGRVYAVSMFNPQGNI MTKGLLTLAKGKPIGKEGYYWLKIHGANCAGVDKVPFPERIKFIEENHENIMACAKSPLENT WWAEQDSPFCFLAFCFEYAGVQHHGLSYNCSLPLAFDGSCSGIQHFSAMLRDEVGGRAVNLL PSETVQDIYGIVAKKVNEILQADAINGTDNEVVTVTDENTGEISEKVKLGTKALAGQWLAYG VTRSVTKRSVMTLAYGSKEFGFRQQVLEDTIQPAIDSGKGLMFTQPNQAAGYMAKLIWESVS VTVVAAVEAMNWLKSAAKLLAAEVKDKKTGEILRKRCAVHWVTPDGFPVWQEYKKPIQTR LNLMFLGQFRLQPTINTNKDSEIDAHKQESGIAPNFVHSQDGSHLRKTVVWAHEKYGIESFALI HDSFGTIPADAANLFKAVRETMVDTYESCDVLADFYDQFADQLHESQLDKMPALPAKGNLNL RDILESDFAFA |

(continued)

| RNA polymerase and variants thereof | Amino acid sequence |
|---|---|
| D438P (SEQ ID NO: 13) | MNTINIAKNDFSDIELAAIPFNTLADHYGERLAREQLALEHESYEMGEARFRKMFERQLKAGE<br>VADNAAAKPLITTLLPKMIARINDWFEEVKAKRGKRPTAFQFLQEIKPEAVAYITIKTTLACLTS<br>ADNTTVQAVASAIGRAIEDEARFGRIRDLEAKHFKKNVEEQLNKRVGHVYKKAFMQVVEAD<br>MLSKGLLGGEAWSSWHKEDSIHVGVRCIEMLIESTGMVSLHRQNAGVVGQDSETIELAPEYA<br>EAIATRAGALAGISPMFQPCVVPPKPWTGITGGGYWANGRRPLALVRTHSKKALMRYEDVYM<br>PEVYKAINIAQNTAWKINKKVLAVANVITKWKHCPVEDIPAIEREELPMKPEDIDMNPEALTAW<br>KRAAAAVYRKDKARKSRRISLEFMLEQANKFANHKAIWFPYNMDWRGRVYAVSMFNPQGNP<br>MTKGLLTLAKGKPIGKEGYYWLKIHGANCAGVDKVPFPERIKFIEENHENIMACAKSPLENT<br>WWAEQDSPFCFLAFCFEYAGVQHHGLSYNCSLPLAFDGSCSGIQHFSAMLRDEVGGRAVNLL<br>PSETVQDIYGIVAKKVNEILQADAINGTDNEVVTVTDENTGEISEKVKLGTKALAGQWLAYG<br>VTRSVTKRSVMTLAYGSKEFGFRQQVLEDTIQPAIDSGKGLMFTQPNQAAGYMAKLIWESVS<br>VTVVAAVEAMNWLKSAAKLLAAEVKDKKTGEILRKRCAVHWVTPDGFPVWQEYKKPIQTR<br>LNLMFLGQFRLQPTINTNKDSEIDAHKQESGIAPNFVHSQDGSHLRKTVVWAHEKYGIESFALI<br>HDSFGTIPADAANLFKAVRETMVDTYESCDVLADFYDQFADQLHESQLDKMPALPAKGNLNL<br>RDILESDFAFA |

(continued)

| RNA polymerase and variants thereof | Amino acid sequence |
|---|---|
| D438V (SEQ ID NO: 14) | MNTINIAKNDFSDIELAAIPFNTLADHYGERLAREQLALEHESYEMGEARFRKMFERQLKAGE<br>VADNAAAKPLITTLLPKMIARINDWFEEVKAKRGKRPTAFQFLQEIKPEAVAYITIKTTLACLTS<br>ADNTTVQAVASAIGRAIEDEARFGRIRDLEAKHFKKNVEEQLNKRVGHVYKKAFMQVVEAD<br>MLSKGLLGGEAWSSWHKEDSIHVGVRCIEMLIESTGMVSLHRQNAGVVGQDSETIELAPEYA<br>EAIATRAGALAGISPMFQPCVVPPKPWTGITGGGYWANGRRPLALVRTHSKKALMRYEDVYM<br>PEVYKAINIAQNTAWKINKKVLAVANVITKWKHCPVEDIPAIEREELPMKPEDIDMNPEALTAW<br>KRAAAAVYRKDKARKSRRISLEFMLEQANKFANHKAIWFPYNMDWRGRVYAVSMFNPQGN<br>VMTKGLLTLAKGKPIGKEGYYWLKIHGANCAGVDKVPFPERIKFIEENHENIMACAKSPLENT<br>WWAEQDSPFCFLAFCFEYAGVQHHGLSYNCSLPLAFDGSCSGIQHFSAMLRDEVGGRAVNLL<br>PSETVQDIYGIVAKKVNEILQADAINGTDNEVVTVTDENTGEISEKVKLGTKALAGQWLAYG<br>VTRSVTKRSVMTLAYGSKEFGFRQQVLEDTIQPAIDSGKGLMFTQPNQAAGYMAKLIWESVS<br>VTVVAAVEAMNWLKSAAKLLAAEVKDKKTGEILRKRCAVHWVTPDGFPVWQEYKKPIQTR<br>LNLMFLGQFRLQPTINTNKDSEIDAHKQESGIAPNFVHSQDGSHLRKTVVWAHEKYGIESFALI<br>HDSFGTIPADAANLFKAVRETMVDTYESCDVLADFYDQFADQLHESQLDKMPALPAKGNLNL<br>RDILESDFAFA |

(continued)

| RNA polymerase and variants thereof | Amino acid sequence |
|---|---|
| R386A+R34A (SEQ ID NO: 15) | MNTINIAKNDFSDIELAAIPFNTLADHYGERLAAEQLALEHESYEMGEARFRKMFERQLKAGE<br>VADNAAAKPLITTLLPKMIARINDWFEEVKAKRGKRPTAFQFLQEIKPEAVAYITIKTTLACLTS<br>ADNTTVQAVASAIGRAIEDEARFGRIRDLEAKHFKKNVEEQLNKRVGHVYKKAFMQVVEAD<br>MLSKGLLGGEAWSSWHKEDSIHVGVRCIEMLIESTGMVSLHRQNAGVVGQDSETIELAPEYA<br>EAIATRAGALAGISPMFQPCVVPPKPWTGITGGGYWANGRRPLALVRTHSKKALMRYEDVYM<br>PEVYKAINIAQNTAWKINKKVLAVANVITKWKHCPVEDIPAIEREELPMKPEDIDMNPEALTAW<br>KRAAAAVYAKDKARKSRRISLEFMLEQANKFANHKAIWFPYNMDWRGRVYAVSMFNPQGN<br>DMTKGLLTLAKGKPIGKEGYYWLKIHGANCAGVDKVPFPERIKFIEENHENIMACAKSPLENT<br>WWAEQDSPFCFLAFCFEYAGVQHHGLSYNCSLPLAFDGSCSGIQHFSAMLRDEVGGRAVNLL<br>PSETVQDIYGIVAKKVNEILQADAINGTDNEVVTVTDENTGEISEKVKLGTKALAGQWLAYG<br>VTRSVTKRSVMTLAYGSKEFGFRQQVLEDTIQPAIDSGKGLMFTQPNQAAGYMAKLIWESVS<br>VTVVAAVEAMNWLKSAAKLLAAEVKDKKTGEILRKRCAVHWVTPDGFPVWQEYKKPIQTR<br>LNLMFLGQFRLQPTINTNKDSEIDAHKQESGIAPNFVHSQDGSHLRKTVVWAHEKYGIESFALI<br>HDSFGTIPADAANLFKAVRETMVDTYESCDVLADFYDQFADQLHESQLDKMPALPAKGNLNL<br>RDILESDFAFA |

(continued)

| RNA polymerase and variants thereof | Amino acid sequence |
|---|---|
| R386M+R34A (SEQ ID NO: 16) | MNTINIAKNDFSDIELAAIPFNTLADHYGERLAAEQLALEHESYEMGEARFRKMFERQLKAGE<br>VADNAAAKPLITTLLPKMIARINDWFEEVKAKRGKRPTAFQFLQEIKPEAVAYITIKTTLACLTS<br>ADNTTVQAVASAIGRAIEDEARFGRIRDLEAKHFKKNVEEQLNKRVGHVYKKAFMQVVEAD<br>MLSKGLLGGEAWSSWHKEDSIHVGVRCIEMLIESTGMVSLHRQNAGVVGQDSETIELAPEYA<br>EAIATRAGALAGISPMFQPCVVPPKPWTGITGGGYWANGRRPLALVRTHSKKALMRYEDVYM<br>PEVYKAINIAQNTAWKINKKVLAVANVITKWKHCPVEDIPAIEREELPMKPEDIDMNPEALTAW<br>KRAAAAVYMKDKARKSRRISLEFMLEQANKFANHKAIWFPYNMDWRGRVYAVSMFNPQGN<br>DMTKGLLTLAKGKPIGKEGYYWLKIHGANCAGVDKVPFPERIKFIEENHENIMACAKSPLENT<br>WWAEQDSPFCFLAFCFEYAGVQHHGLSYNCSLPLAFDGSCSGIQHFSAMLRDEVGGRAVNLL<br>PSETVQDIYGIVAKKVNEILQADAINGTDNEVVTVTDENTGEISEKVKLGTKALAGQWLAYG<br>VTRSVTKRSVMTLAYGSKEFGFRQQVLEDTIQPAIDSGKGLMFTQPNQAAGYMAKLIWESVS<br>VTVVAAVEAMNWLKSAAKLLAAEVKDKKTGEILRKRCAVHWVTPDGFPVWQEYKKPIQTR<br>LNLMFLGQFRLQPTINTNKDSEIDAHKQESGIAPNFVHSQDGSHLRKTVVWAHEKYGIESFALI<br>HDSFGTIPADAANLFKAVRETMVDTYESCDVLADFYDQFADQLHESQLDKMPALPAKGNLNL<br>RDILESDFAFA |

(continued)

| RNA polymerase and variants thereof | Amino acid sequence |
|---|---|
| R386M+Y178H (SEQ ID NO: 17) | MNTINIAKNDFSDIELAAIPFNTLADHYGERLAREQLALEHESYEMGEARFRKMFERQLKAGE<br>VADNAAAKPLITTLLPKMIARINDWFEEVKAKRGKRPTAFQFLQEIKPEAVAYITIKTTLACLTS<br>ADNTTVQAVASAIGRAIEDEARFGRIRDLEAKHFKKNVEEQLNKRVGHVHKKAFMQVVEAD<br>MLSKGLLGGEAWSSWHKEDSIHVGVRCIEMLIESTGMVSLHRQNAGVVGQDSETIELAPEYA<br>EAIATRAGALAGISPMFQPCVVPPKPWTGITGGGYWANGRRPLALVRTHSKKALMRYEDVYM<br>PEVYKAINIAQNTAWKINKKVLAVANVITKWKHCPVEDIPAIEREELPMKPEDIDMNPEALTAW<br>KRAAAAVYMKDKARKSRRISLEFMLEQANKFANHKAIWFPYNMDWRGRVYAVSMFNPQGN<br>DMTKGLLTLAKGKPIGKEGYYWLKIHGANCAGVDKVPFPERIKFIEENHENIMACAKSPLENT<br>WWAEQDSPFCFLAFCFEYAGVQHHGLSYNCSLPLAFDGSCSGIQHFSAMLRDEVGGRAVNLL<br>PSETVQDIYGIVAKKVNEILQADAINGTDNEVVTVTDENTGEISEKVKLGTKALAGQWLAYG<br>VTRSVTKRSVMTLAYGSKEFGFRQQVLEDTIQPAIDSGKGLMFTQPNQAAGYMAKLIWESVS<br>VTVVAAVEAMNWLKSAAKLLAAEVKDKKTGEILRKRCAVHWVTPDGFPVWQEYKKPIQTR<br>LNLMFLGQFRLQPTINTNKDSEIDAHKQESGIAPNFVHSQDGSHLRKTVVWAHEKYGIESFALI<br>HDSFGTIPADAANLFKAVRETMVDTYESCDVLADFYDQFADQLHESQLDKMPALPAKGNLNL<br>RDILESDFAFA |

(continued)

| RNA polymerase and variants thereof | Amino acid sequence |
| --- | --- |
| R386W+N437T (SEQ ID NO: 18) | MNTINIAKNDFSDIELAAIPFNTLADHYGERLAREQLALEHESYEMGEARFRKMFERQLKAGE<br>VADNAAAKPLITTLLPKMIARINDWFEEVKAKRGKRPTAFQFLQEIKPEAVAYITIKTTLACLTS<br>ADNTTVQAVASAIGRAIEDEARFGRIRDLEAKHFKKNVEEQLNKRVGHVYKKAFMQVVEAD<br>MLSKGLLGGEAWSSWHKEDSIHVGVRCIEMLIESTGMVSLHRQNAGVVGQDSETIELAPEYA<br>EAIATRAGALAGISPMFQPCVVPPKPWTGITGGGYWANGRRPLALVRTHSKKALMRYEDVYM<br>PEVYKAINIAQNTAWKINKKVLAVANVITKWKHCPVEDIPAIEREELPMKPEDIDMNPEALTAW<br>KRAAAAVYWKDKARKSRRISLEFMLEQANKFANHKAIWFPYNMDWRGRVYAVSMFNPQGT<br>DMTKGLLTLAKGKPIGKEGYYWLKIHGANCAGVDKVPFPERIKFIEENHENIMACAKSPLENT<br>WWAEQDSPFCFLAFCFEYAGVQHHGLSYNCSLPLAFDGSCSGIQHFSAMLRDEVGGRAVNLL<br>PSETVQDIYGIVAKKVNEILQADAINGTDNEVVTVTDENTGEISEKVKLGTKALAGQWLAYG<br>VTRSVTKRSVMTLAYGSKEFGFRQQVLEDTIQPAIDSGKGLMFTQPNQAAGYMAKLIWESVS<br>VTVVAAVEAMNWLKSAAKLLAAEVKDKKTGEILRKRCAVHWVTPDGFPVWQEYKKPIQTR<br>LNLMFLGQFRLQPTINTNKDSEIDAHKQESGIAPNFVHSQDGSHLRKTVVWAHEKYGIESFALI<br>HDSFGTIPADAANLFKAVRETMVDTYESCDVLADFYDQFADQLHESQLDKMPALPAKGNLNL<br>RDILESDFAFA |

(continued)

| RNA polymerase and variants thereof | Amino acid sequence |
| --- | --- |
| R386I+Del172 (SEQ ID NO: 19) | MNTINIAKNDFSDIELAAIPFNTLADHYGERLAREQLALEHESYEMGEARFRKMFERQLKAGE VADNAAAKPLITTLLPKMIARINDWFEEVKAKRGKRPTAFQFLQEIKPEAVAYITIKTTLACLTS ADNTTVQAVASAIGRAIEDEARFGRIRDLEAKHFKKNVEEQLNRVGHVYKKAFMQVVEADM LSKGLLGGEAWSSWHKEDSIHVGVRCIEMLIESTGMVSLHRQNAGVVGQDSETIELAPEYAE AIATRAGALAGISPMFQPCVVPPKPWTGITGGGYWANGRRPLALVRTHSKKALMRYEDVYMP EVYKAINIAQNTAWKINKKVLAVANVITKWKHCPVEDIPAIEREELPMKPEDIDMNPEALTAW KRAAAAVYIKDKARKSRRISLEFMLEQANKFANHKAIWFPYNMDWRGRVYAVSMFNPQGND MTKGLLTLAKGKPIGKEGYYWLKIHGANCAGVDKVPFPERIKFIEENHENIMACAKSPLENT WWAEQDSPFCFLAFCFEYAGVQHHGLSYNCSLPLAFDGSCSGIQHFSAMLRDEVGGRAVNLL PSETVQDIYGIVAKKVNEILQADAINGTDNEVVTVTDENTGEISEKVKLGTKALAGQWLAYG VTRSVTKRSVMTLAYGSKEFGFRQQVLEDTIQPAIDSGKGLMFTQPNQAAGYMAKLIWESVS VTVVAAVEAMNWLKSAAKLLAAEVKDKKTGEILRKRCAVHWVTPDGFPVWQEYKKPIQTR LNLMFLGQFRLQPTINTNKDSEIDAHKQESGIAPNFVHSQDGSHLRKTVVWAHEKYGIESFALI HDSFGTIPADAANLFKAVRETMVDTYESCDVLADFYDQFADQLHESQLDKMPALPAKGNLNL RDILESDFAFA |

(continued)

| RNA polymerase and variants thereof | Amino acid sequence |
|---|---|
| R386F+Y178D (SEQ ID NO: 20) | MNTINIAKNDFSDIELAAIPFNTLADHYGERLAREQLALEHESYEMGEARFRKMFERQLKAGE<br>VADNAAAKPLITTLLPKMIARINDWFEEVKAKRGKRPTAFQFLQEIKPEAVAYITIKTTLACLTS<br>ADNTTVQAVASAIGRAIEDEARFGRIRDLEAKHFKKNVEEQLNKRVGHVDKKAFMQVVEAD<br>MLSKGLLGGEAWSSWHKEDSIHVGVRCIEMLIESTGMVSLHRQNAGVVGQDSETIELAPEYA<br>EAIATRAGALAGISPMFQPCVVPPKPWTGITGGGYWANGRRPLALVRTHSKKALMRYEDVYM<br>PEVYKAINIAQNTAWKINKKVLAVANVITKWKHCPVEDIPAIEREELPMKPEDIDMNPEALTAW<br>KRAAAAVYFKDKARKSRRISLEFMLEQANKFANHKAIWFPYNMDWRGRVYAVSMFNPQGN<br>DMTKGLLTLAKGKPIGKEGYYWLKIHGANCAGVDKVPFPERIKFIEENHENIMACAKSPLENT<br>WWAEQDSPFCFLAFCFEYAGVQHHGLSYNCSLPLAFDGSCSGIQHFSAMLRDEVGGRAVNLL<br>PSETVQDIYGIVAKKVNEILQADAINGTDNEVVTVTDENTGEISEKVKLGTKALAGQWLAYG<br>VTRSVTKRSVMTLAYGSKEFGFRQQVLEDTIQPAIDSGKGLMFTQPNQAAGYMAKLIWESVS<br>VTVVAAVEAMNWLKSAAKLLAAEVKDKKTGEILRKRCAVHWVTPDGFPVWQEYKKPIQTR<br>LNLMFLGQFRLQPTINTNKDSEIDAHKQESGIAPNFVHSQDGSHLRKTVVWAHEKYGIESFALI<br>HDSFGTIPADAANLFKAVRETMVDTYESCDVLADFYDQFADQLHESQLDKMPALPAKGNLNL<br>RDILESDFAFA |

(continued)

| RNA polymerase and variants thereof | Amino acid sequence |
|---|---|
| D388K+D438P (SEQ ID NO: 21) | MNTINIAKNDFSDIELAAIPFNTLADHYGERLAREQLALEHESYEMGEARFRKMFERQLKAGE VADNAAAKPLITTLLPKMIARINDWFEEVKAKRGKRPTAFQFLQEIKPEAVAYITIKTTLACLTS ADNTTVQAVASAIGRAIEDEARFGRIRDLEAKHFKKNVEEQLNKRVGHVYKKAFMQVVEAD MLSKGLLGGEAWSSWHKEDSIHVGVRCIEMLIESTGMVSLHRQNAGVVGQDSETIELAPEYA EAIATRAGALAGISPMFQPCVVPPKPWTGITGGGYWANGRRPLALVRTHSKKALMRYEDVYM PEVYKAINIAQNTAWKINKKVLAVANVITKWKHCPVEDIPAIEREELPMKPEDIDMNPEALTAW KRAAAAVYRKKKARKSRRISLEFMLEQANKFANHKAIWFPYNMDWRGRVYAVSMFNPQGNP MTKGLLTLAKGKPIGKEGYYWLKIHGANCAGVDKVPFPERIKFIEENHENIMACAKSPLENT WWAEQDSPFCFLAFCFEYAGVQHHGLSYNCSLPLAFDGSCSGIQHFSAMLRDEVGGRAVNLL PSETVQDIYGIVAKKVNEILQADAINGTDNEVVTVTDENTGEISEKVKLGTKALAGQWLAYG VTRSVTKRSVMTLAYGSKEFGFRQQVLEDTIQPAIDSGKGLMFTQPNQAAGYMAKLIWESVS VTVVAAVEAMNWLKSAAKLLAAEVKDKKTGEILRKRCAVHWVTPDGFPVWQEYKKPIQTR LNLMFLGQFRLQPTINTNKDSEIDAHKQESGIAPNFVHSQDGSHLRKTVVWAHEKYGIESFALI HDSFGTIPADAANLFKAVRETMVDTYESCDVLADFYDQFADQLHESQLDKMPALPAKGNLNL RDILESDFAFA |

(continued)

| RNA polymerase and variants thereof | Amino acid sequence |
|---|---|
| R386C+D438P (SEQ ID NO: 22) | MNTINIAKNDFSDIELAAIPFNTLADHYGERLAREQLALEHESYEMGEARFRKMFERQLKAGE<br>VADNAAAKPLITTLLPKMIARINDWFEEVKAKRGKRPTAFQFLQEIKPEAVAYITIKTTLACLTS<br>ADNTTVQAVASAIGRAIEDEARFGRIRDLEAKHFKKNVEEQLNKRVGHVYKKAFMQVVEAD<br>MLSKGLLGGEAWSSWHKEDSIHVGVRCIEMLIESTGMVSLHRQNAGVVGQDSETIELAPEYA<br>EAIATRAGALAGISPMFQPCVVPPKPWTGITGGGYWANGRRPLALVRTHSKKALMRYEDVYM<br>PEVYKAINIAQNTAWKINKKVLAVANVITKWKHCPVEDIPAIEREELPMKPEDIDMNPEALTAW<br>KRAAAAVYCKDKARKSRRISLEFMLEQANKFANHKAIWFPYNMDWRGRVYAVSMFNPQGNP<br>MTKGLLTLAKGKPIGKEGYYWLKIHGANCAGVDKVPFPERIKFIEENHENIMACAKSPLENT<br>WWAEQDSPFCFLAFCFEYAGVQHHGLSYNCSLPLAFDGSCSGIQHFSAMLRDEVGGRAVNLL<br>PSETVQDIYGIVAKKVNEILQADAINGTDNEVVTVTDENTGEISEKVKLGTKALAGQWLAYG<br>VTRSVTKRSVMTLAYGSKEFGFRQQVLEDTIQPAIDSGKGLMFTQPNQAAGYMAKLIWESVS<br>VTVVAAVEAMNWLKSAAKLLAAEVKDKKTGEILRKRCAVHWVTPDGFPVWQEYKKPIQTR<br>LNLMFLGQFRLQPTINTNKDSEIDAHKQESGIAPNFVHSQDGSHLRKTVVWAHEKYGIESFALI<br>HDSFGTIPADAANLFKAVRETMVDTYESCDVLADFYDQFADQLHESQLDKMPALPAKGNLNL<br>RDILESDFAFA |

**Example 2 Preparation of mRNA by In-Vitro Transcription Reaction**

An enzyme stock solution was diluted with a storage buffer (50 mM Tris-HCl (25°C, pH 7.9), 100 mM NaCl, 0.1 mM EDTA, 2 mM DTT, 0.1% Triton X-100, 50% Glycerol) to achieve an enzyme activity of 400 U/μL. A MIX solution was prepared in an EP tube according to the reaction system (20 μL) in Table 2. Then, the MIX solution was transferred to an eight-tube strip, mixed uniformly, and then centrifuged. The eight-tube strip was placed in a PCR machine for reaction at 37°C for 1 h. Then, 36 μL of magnetic beads (Vazyme, Cat. No.: N412) was added, mixed uniformly, and then incubated at room temperature for 2-5 min. The mixture was placed on a magnetic rack for mRNA purification and then transferred to an RNase-free centrifuge tube to obtain the purified mRNA.

Table 2: Reaction system composition

| Component | Stock solution concentration | Volume (μL) |
|---|---|---|
| ATP | 100 mM | 1.2 |
| GTP | 100 mM | 1.2 |
| CTP | 100 mM | 1.2 |
| m1ψTP | 100 mM | 1.2 |
| RNase inhibitor (Vazyme, Cat. No.: GMP4102PA) | 40 U/μL | 1 |
| Inorganic pyrophosphatase (Vazyme, Cat. No.: GMP4103PC) | 0.1 U/μL | 1 |
| CAG Trimer (Vazyme, Cat. No.: GMP4118PC-01) | 100 mM | 0.4 |
| Template DNA (SEQ ID NO: 45 (sense strand)) | \ | 1 μg |
| 10× transcription buffer (Hepes buffer, 20 mM Spermidine, 25 mM TCEP, pH 7.01) | 400 mM | 2 |
| $MgCl_2$ | 200 mM | 1.2 |
| T7 RNA polymerase | 400 U/μL | 1 |
| $ddH_2O$ | \ | Fill up to 20 μL |

**Example 3 Capping Rate Determination**

After pre-treatment with the mRNA Capping Rate Assay Kit (Vazyme, Cat. No.: DD3510-01), the capping rates of the mRNA products were determined by LC-MS.
(1) The purified mRNA of Example 2 was conjugated to a probe. The reaction system is shown in Table 3, and the reaction conditions are shown in Table 4.

Table 3: Reaction system

| **Component** | Volume |
|---|---|
| mRNA | 25 pmol (V) |
| RNase-free $H_2O$ | (20-***V***) μL |
| Probe (50 μM) (Genscript Custom) | 1 μL |
| Total | 21 μL |

Table 4: Reaction condition

| **Temperature** | Time |
|---|---|
| 95°C | 2 mins |
| 70°C-16°C | -0.1 °C/s, ≈ 180 cycles |
| 16°C | ∞ |

(2) RNase H cleavage: The cleavage reaction system was prepared according to Table 5, mixed uniformly by vortexing, and then placed in a PCR machine for reaction at 25°C for 20 min.

Table 5: Reaction system

| **Component** | Volume |
|---|---|
| Product from the previous step | 21 μL |
| RNase H Reaction Buffer (10×) | 3 μL |
| RNase H (5 U/μl) | 3 μL |
| RNase-free $H_2O$ | 3 μL |
| Total | 30 μL |

(3) SA magnetic bead binding:

a) Magnetic bead washing: 9 μL of SA magnetic beads (Cat. No.: SM017005) were taken into a centrifuge tube and placed on a magnetic rack. After the solution became clear, the supernatant was aspirated and discarded using a pipette. The centrifuge tube was removed from the magnetic rack. 200 μL of RNase-free $H_2O$ was added for rinsing, and the tube was then placed back on the magnetic rack. After the solution became clear, the supernatant was aspirated and discarded using a pipette. Then, 200 μL of RNase-free H2O was added for rinsing once again.
b) Reaction conditions: The centrifuge tube was removed from the magnetic rack, and the cleaved product was added to the SA magnetic beads (solid). The mixture was pipetted 20-30 times using a pipette to ensure thorough and uniform mixing, and then placed on a rotator and incubated under rotating at room temperature for 30 min to allow the magnetic beads to fully bind to the cleaved product.

(4) Rinsing and elution:

a) The product from the previous step was placed on the magnetic rack for 2-3 min. After the solution became clear, the supernatant was aspirated and discarded using a pipette.
b) 200 μL of rinse buffer was added for rinsing, taking care not to disturb the magnetic beads. The mixture was left to stand for 0.5-1 min, and then the supernatant was aspirated and discarded using a pipette.
c) Step b) was repeated.
d) The centrifuge tube was removed from the magnetic rack, and 30 μL of elution buffer was added. The mixture was mixed uniformly by pipetting 10-20 times using a pipette, so that the magnetic beads were uniformly dispersed, thereby ensuring sufficient elution.
e) The tube was placed in a PCR machine. After reaction at 85°C for 3 min, the tube was placed on the magnetic rack immediately. After the solution became clear (0.5-1 min), the supernatant was aspirated into a new centrifuge tube. The supernatant was the desired product.
f) The above product was sent to Thermo scientific Vanquish Flex-Qrbitrap Exploris120 to determine the capping rate (mobile phase: phase A: 2% hexafluoroisopropanol - 1% N',N-diisopropylethylamine in water, and phase B: 2% hexafluoroisopropanol - 1% N',N-diisopropylethylamine in methanol; chromatographic column: Nano ChromCore C18 3 μm, 4.6*100 mm; ion mode: negative ion mode; scanning mode: Full scan; scanning range: 600-3000). The capping rate was calculated according to the formula:

$$\text{mRNA capping rate (\%)} = (\text{capped mRNA} /(\text{capped mRNA} + \text{uncapped mRNA})) \times 100\%.$$

The results are as shown in FIG. 4. When the ratio of the cap analogue to raw material NTP was as low as 0.33 : 1, the T7 RNA polymerase variants in Example 1 were all able to well improve the capping rate of the mRNA products. R386W+N437T increased the capping rate to 100%.

**Example 4 Preparation of mRNA by In-Vitro Transcription Reaction**

mRNA was prepared by in-vitro transcription with reference to Example 2 above, in which the amount of cap analogue added was further reduced to 0.24 μL (specifically as shown in Table 6), and the remaining reaction conditions were the same. Further, the capping rate was determined with reference to Example 3.

Table 6: Reaction system composition

| Component | Stock solution concentration | Volume (μL) |
|---|---|---|
| ATP | 100 mM | 1.2 |
| GTP | 100 mM | 1.2 |
| CTP | 100 mM | 1.2 |
| m1ψTP | 100 mM | 1.2 |
| RNase inhibitor (Vazyme, Cat. No.: GMP4102PA) | 40 U/μL | 1 |
| Inorganic pyrophosphatase (Vazyme, Cat. No.: GMP4103PC) | 0.1 U/μL | 1 |
| CAG Trimer (Vazyme, Cat. No.: GMP4118PC-01) | 100 mM | 0.24 |
| Template DNA (SEQ ID NO: 45 (sense strand)) | \ | 1 μg |
| 10× transcription buffer (Hepes buffer, 20 mM Spermidine, 25 mM TCEP, pH 7.01) | 400 mM | 2 |
| $MgCl_2$ | 200 mM | 1.2 |
| T7 RNA polymerase | 400 U/μL | 1 |
| $ddH_2O$ | \ | Fill up to 20 μL |

The results are as shown in FIG. 5. All mutation sites effectively increased the capping rate relative to T7 RNAP wild-type. The combinatorial mutations R386C+D438P, R386W+N437T and D388K+D438P in T7 RNAP exhibited a further significant increase in the capping rate on the basis of single-site mutations. Among them, D388K+D438P increased the capping rate to 95.5% relative to the capping rate of 84% for D388K and 68.5% for D438P, with an increase amplitude of > 10%.

## Claims

1. An RNA polymerase variant, **characterised in that** the amino acid sequence of the variant comprises at least one substitution or deletion at amino acid positions selected from R386, R34, K172, Y178, D388, Q435, N437 or D438, relative to SEQ ID NO: 1.

2. The variant according to claim 1, **characterised in that**

   (1) the substitution at position R386 is selected from C or W;
   (2) the substitution at position D388 is K;
   (3) the substitution at position Q435 is selected from A, H or T;
   (4) the substitution at position N437 is selected from F or T;
   (5) the substitution at position D438 is selected from T, L, I, P or V;
   (6) the substitution at position R34 is selected from A;
   (7) the mutation at position K172 is a deletion; and
   (8) the substitution at position Y178 is selected from H or D.

3. The variant according to claim 1 or 2, **characterised in that** the amino acid sequence of the variant comprises any one of mutation at positions selected from R386W, R386C, D388K, Q435A, Q435H, Q435T, N437F, N437T, D438T, D438L, D438I, D438P, D438V, R386A+R34A, R386M+R34A, R386M+Y178H, R386W+N437T, R386I+del-K172, R386F+Y178D, D388K+D438P or R386C+D438P, relative to SEQ ID NO: 1.

4. The variant according to any one of claims 1-3, **characterised in that** the variant has an amino acid sequence as set forth in any one of SEQ ID NOs: 2-22.

5. A polynucleotide molecule, **characterised in that** the polynucleotide molecule encodes the variant according to any one of claims 1-4.

6. An expression vector, **characterised in that** the expression vector comprises the polynucleotide molecule according

to claim 5.

**7.** A host cell, **characterised in that** the host cell comprises the polynucleotide molecule according to claim 5 or the expression vector according to claim 6.

**8.** A method for preparing the variant according to any one of claims 1-4, **characterised in that** the method comprises:

culturing the host cell according to claim 7; and
recovering the variant.

**9.** A composition, **characterised in that** the composition comprises the variant according to any one of claims 1-4.

**10.** A kit, **characterised in that** the kit comprises the variant according to any one of claims 1-4.

**11.** Use of the variant according to any one of claims 1-4, the composition according to claim 9, or the kit according to claim 10 in *in-vitro* transcription.

**12.** A method for preparing RNA, **characterised in that** the method comprises contacting a DNA template and a modified or unmodified nucleoside triphosphate with the RNA polymerase variant according to any one of claims 1-4, and performing an incubation in an *in-vitro* transcription reaction system to obtain an RNA product of interest.

**13.** A method for preparing capped mRNA, **characterised in that** the method comprises contacting a DNA template, a modified or unmodified nucleoside triphosphate and a cap analogue with the RNA polymerase variant according to any one of claims 1-4, and performing an incubation in an *in-vitro* transcription reaction system to obtain a product of interest.

**14.** The method according to claim 13, **characterised in that** the cap analogue is a trinucleotide cap.

**15.** The method according to claim 14, **characterised in that** the trinucleotide cap is m7GpppA2'OMepG.

**16.** The method according to any one of claims 12-15, **characterised in that** the DNA template has 2'-deoxyguanosine residues at positions + 1 and + 2.

**17.** Use of the variant according to any one of claims 1-4, the composition according to claim 9, or the kit according to claim 10 in transcription of a DNA template having 2'-deoxyguanosine residues at positions + 1 and + 2 of the template.

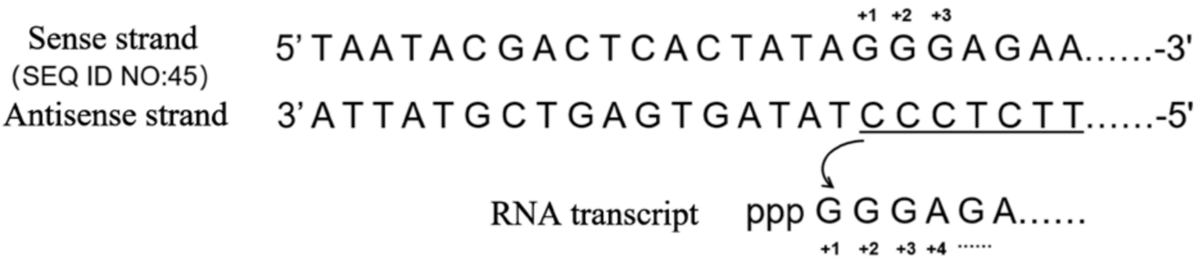


FIG. 1

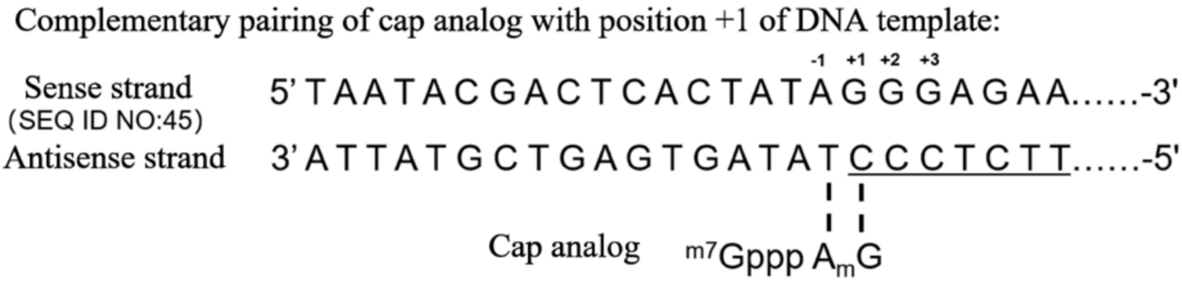


FIG. 2

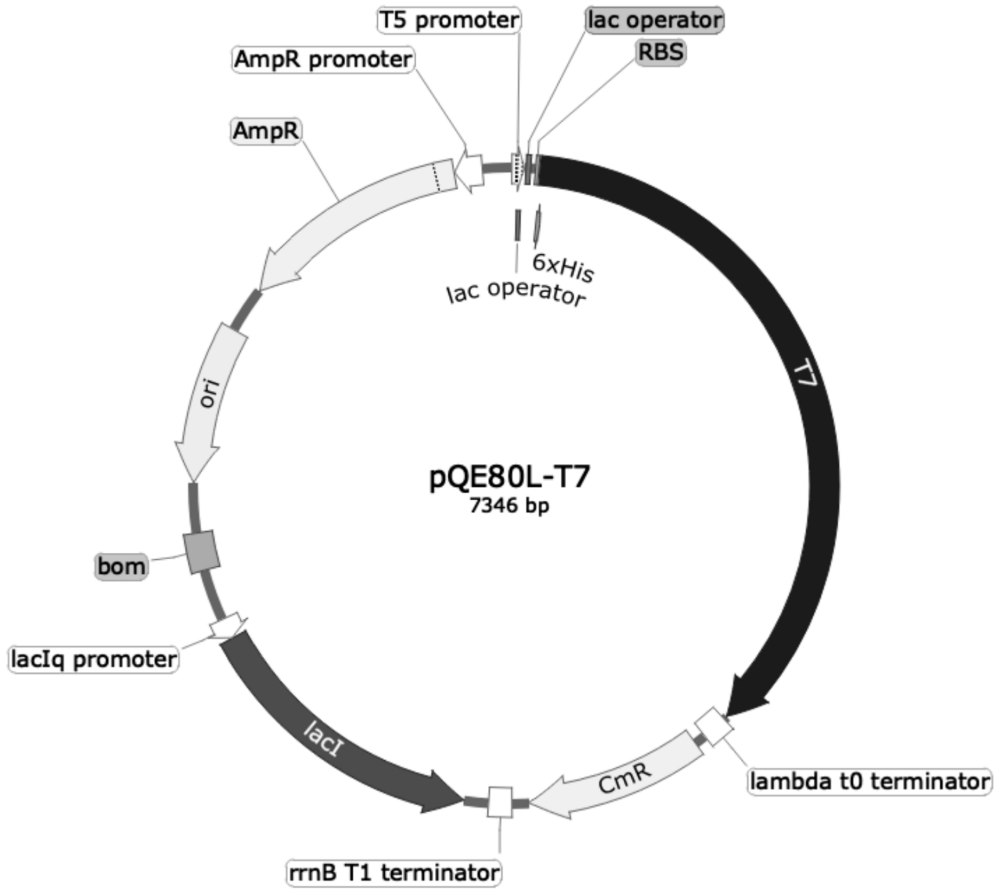


FIG. 3

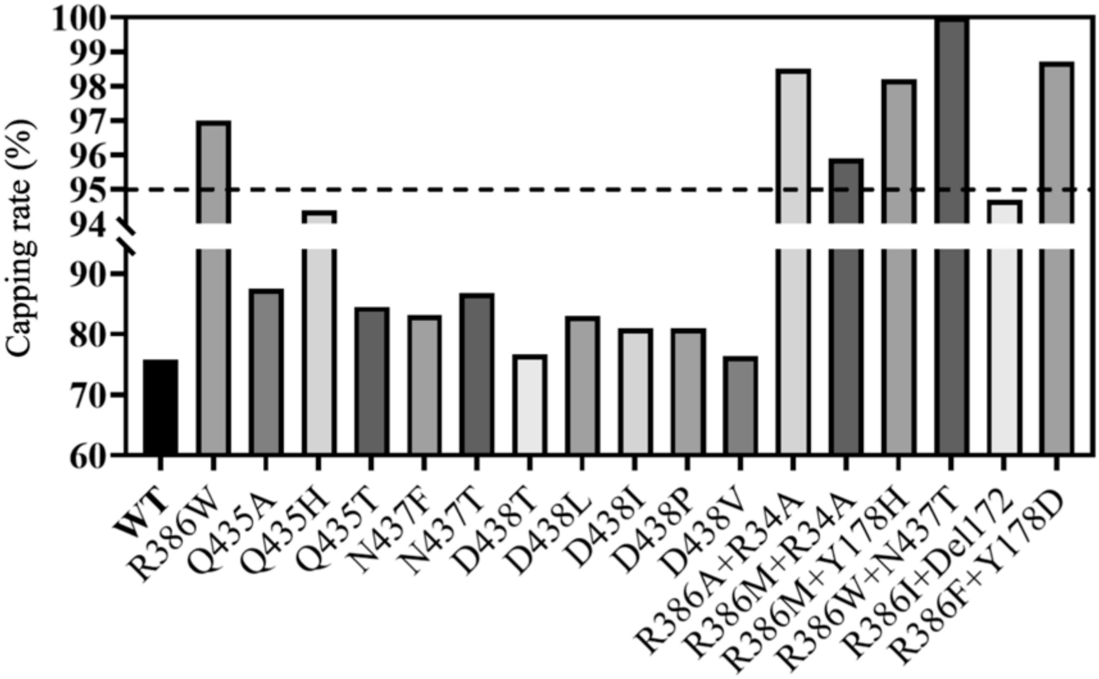
Capping rate (%)
100
99
98
97
96
95
94
90
80
70
60
WT
R386W
Q435A
Q435H
Q435T
N437F
N437T
D438T
D438L
D438I
D438P
D438V
R386A+R34A
R386M+R34A
R386M+Y178H
R386W+N437T
R386I+Del172
R386F+Y178D

FIG. 4

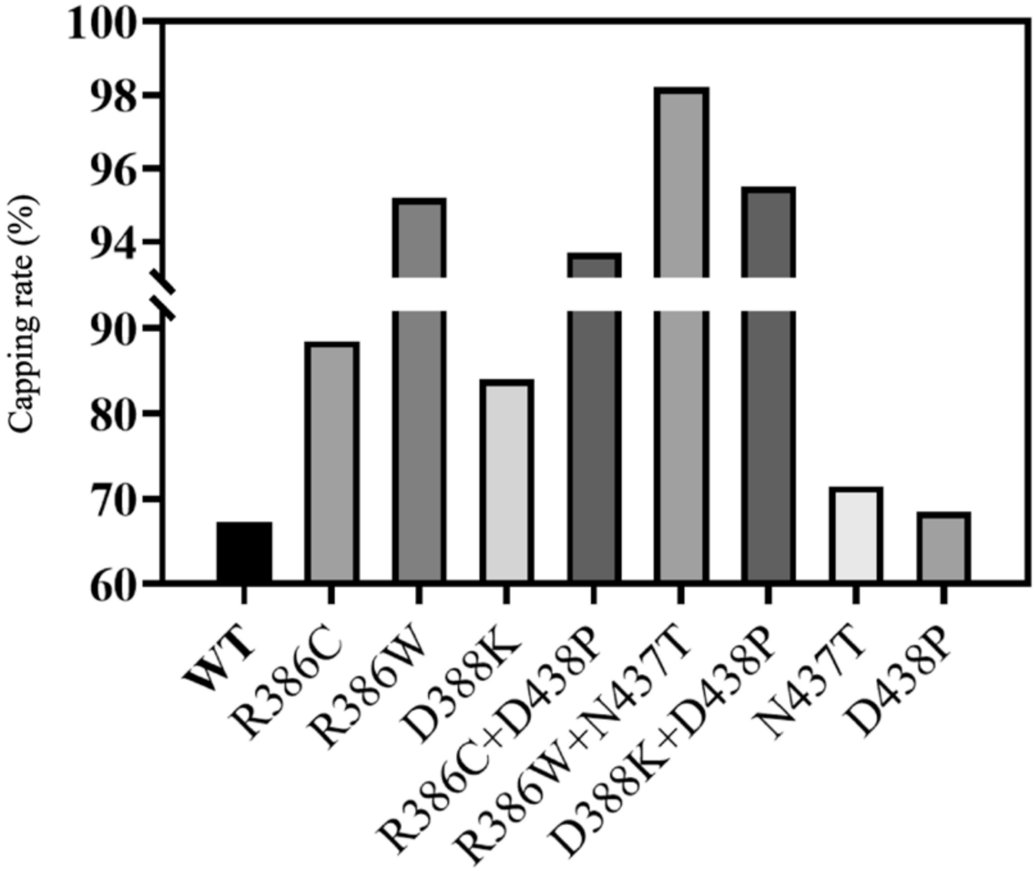
Capping rate (%)
100
98
96
94
90
80
70
60
WT
R386C
R386W
D388K
R386C+D438P
R386W+N437T
D388K+D438P
N437T
D438P

FIG. 5